# EUROPÄISCHE PATENTANMELDUNG

(11) **EP 0 722 691 A1**
(43) Veröffentlichungstag der Anmeldung: **24.07.1996**
(21) Anmeldenummer: 95120096.3
(22) Anmeldetag: 19.12.1995
(51) Int. Cl.: A61B 5/00

(54) **System zur Bestimmung von Gewebeeigenschaften**

(30) Priorität: 24.12.1994 DE 4446721; 22.06.1995 DE 19522706
(71) Anmelder: BOEHRINGER MANNHEIM GMBH, D-68298 Mannheim (DE)
(72) Erfinder: Röper, Josef, Dr., D-67141 Neuhofen (DE); Böcker, Dirk, Dr. Dr., D-69115 Heidelberg (DE)

(57) **Zusammenfassung**

System zur Bestimmung von Eigenschaften von Gewebeteilen eines lebenden Organismus aufgebaut aus einer optoelektronischen Meßvorrichtung (10) und einer Befestigungsvorrichtung (20), die für Licht undurchlässig ist, und die ein Meßfenster (28) umgibt, durch welches die Bestimmung vorgenommen werden kann und wobei die Vorrichtung (20) ein oder mehrere Bauelemente (26) zur reproduzierbaren auf eine erste Obenfläche des Gewebes gerichteten Fixierung der Meßvorrichtung (10) aufweist. Ebenfalls Gegenstand der Erfindung ist die Befestigungsvorrichtung (20) sowie ein Verfahren zur Bestimmung von Eigenschaften von Gewebeteilen mit Hilfes des beanspruchten Systems.

## Beschreibung

Gegenstand der Erfindung ist ein System zur Bestimmung von Eigenschaften von Gewebeteilen eines lebenden Organismus, eine Vorrichtung zur Befestigung einer optoelektronischen Meßvorrichtung an der Oberfläche des Gewebes sowie ein Verfahren zur nicht-invasiven Bestimmung von Eigenschaften von Gewebeteilen.

Verfahren zur Bestimmung von Eigenschaften von lebenden Organismen wurden bisher in der überwiegenden Anzahl der Fälle invasiv durchgeführt. So wurden beispielsweise Strukturen von Geweben durch chirurgische Eingriffe charakterisiert. Für die Bestimmung von Analyten in Körperflüssigkeiten wurde dem Gewebe, z. B. dem menschlichen Körper, eine gewisse Menge der Körperflüssigkeit entzogen, z. B. durch Blutentnahme aus den Venen. Solche invasiven Verfahren führten, insbesondere wenn die Bestimmungen mehrmals hintereinander durchgeführt werden mußten, zu Schädigungen des Gewebes. Aus diesem Grund wurden in jüngerer Zeit Verfahren entwickelt, mit denen auch ohne Entnahme von Körperflüssigkeiten oder chirurgische Eingriffe Eigenschaften von Gewebeteilen eines lebenden Organismus bestimmt werden können.

In WO 94/10901 ist ein Verfahren und eine Vorrichtung zur Analyse von Glukose in einer biologischen Matrix beschrieben, welches die Mehrfachstreuung von Licht in Gewebe ausnutzt. In dieser Patentanmeldung ist beschrieben, daß ein Meßkopf mit Hilfe eines Klebebandes an einer geeigneten Steile des Körpers befestigt wird. Um Fremdlicht abzuhalten weist der Meßkopf ein Hautkontaktteil auf, dessen Durchmesser wesentlich größer als der der verwendeten Meßöffnung ist. Der Ring besteht hierzu aus einem undurchsichtigen Material und schließt mit der Haut ab.

In US-A-5,226,417 ist eine Vorrichtung beschrieben, mit der ein Finger durchstrahlt werden kann. Hierzu weist die Vorrichtung lichtemittierende Dioden und Photodektoren auf, die flexibel miteinander verbunden sind, so daß sie auf gegenüberliegenden Seiten des Fingers angeordnet werden können. Die Vorrichtung enthält außerdem Flügel, die bei Anlegen der Vorrichtung an einen Finger eine Abschirmung von Umgebungslicht bewirken sollen.

Darüber hinaus enthält die beschriebene Vorrichtung ein Klebeband zur Befestigung der Lichtquelle und des Photodetektors auf der Haut. Zweck der Verwendung des Klebers ist die Resistenz gegen unbeabsichtigte Entfernung der Vorrichtung.

In US-A-4,928,691 ist ebenfalls eine flexible Vorrichtung beschrieben, die einen Photosensor und eine Lichtquelle enthält, wobei diese Elemente auf einem biegsamen Material befestigt sind, welches an unterschiedlichen Körperstellen befestigt werden kann. Die Befestigung geschieht mit Hilfe eines Klebestreifens.

In US-A-5,267,563 ist ein Pulsoximeter beschrieben, dessen Sensoren mit Hilfe eines Klebepflasters auf der Haut befestigt werden. Das Pflaster enthält Stoffe zur Anregung der Durchblutung. Wenn das Arzneimittel in die Haut eingedrungen ist, wird die Messung durchgeführt. Anschließend wird das Meßgerät wieder von der Haut entfernt.

In EP-A-0 573 137 ist ebenfalls ein Pulsoximeter beschrieben, wobei dessen Sensoren mittels eines Klebestreifens, der die Durchblutung anregende Stoffe enthält, auf der Haut befestigt werden. Zur Abschirmung von Umgebungslicht ist der Photodetektor von einer Wandung umgeben, die durch den Kleber bis auf die Haut reicht. Wie die vorstehend genannten Vorrichtungen ist diese für den einmaligen Gebrauch vorgesehen.

In US-A-4,685,464 ist ein Element zur Befestigung eines Sensors zur nicht-invasiven Messung von Blutbestandteilen beschrieben, der zur Aufnahme eines Fingers geeignet ist.

In EP-A-0 572 684 ist ein Sensor zum Monitoring von Lebenszeichen, insbesondere der Sauerstoffsättigung, beschrieben, der an verschiedenen Stellen der Haut befestigt werden kann. Hierzu enthält das beschriebene System ein Transmitterelement sowie ein Empfängerelement, welche für mehrfache Verwendung ausgelegt sind. Ferner enthält das System ein Gehäuse zur Unterbringung der oben genannten Elemente und Verbinder zur Befestigung der oben genannten Elemente.

Bei den im oben genannten Stand der Technik beschriebenen Vorrichtungen und Verfahren kommt es auf eine Positioniergenauigkeit nicht an, da die Anwendung in der Pulsoximetrie nur vom Pulssignal, nicht vom Ort abhängig ist.

In US-A-4,798,955 ist ein Hilfsmittel zur Verwendung eines nicht-invasiven Meßgerätes für mehrere Personen beschrieben. Es ist darin ein flexibles Band beschrieben, welches um den Oberarm der Personen gelegt wird. Dieses Band weist ein Element auf, welches zur individuellen Justierung des Bandes und somit eines Meßfensters an den jeweiligen Personen geeignet ist. Es hat sich jedoch gezeigt, daß die Reproduzierbarkeit der individuellen Meßergebnisse auch hiermit nicht befriedigend ist.

Aufgabe der vorliegenden Erfindung war es, die Nachteile des Standes der Technik vollständig oder zumindest teilweise zu beseitigen.

Insbesondere war es Aufgabe der Erfindung, ein zur Bestimmung von Gewebeeigenschaften geeignetes System zur Verfügung zu stellen, welches auch für die Langzeitanwendung geeignet ist.

Diese Aufgabe wird dadurch gelöst, daß eine Meßvorrichtung positionsgenau an der Oberfläche des Gewebes fixiert wird.

Gegenstand der Erfindung ist daher ein System zur Bestimmung von Eigenschaften von Gewebeteilen eines lebenden Organismus enthaltend
- eine optoelektronische Meßvorrichtung (10),
- eine Vorrichtung (20), die für Licht undurchlässig ist, und die ein Meßfenster (28) umgibt, durch welches die Bestimmung vorgenommen werden kann, und wobei die Vorrichtung mindestens ein Bauelement (26) zur reproduzierbaren, auf eine erste Oberfläche des Gewebes gerichteten, Fixierung der Meßvorrichtung (10) aufweist.

Außerdem sind Gegenstände der Erfindung eine Vorrichtung zur Befestigung einer optoelektronischen Meßvorrichtung an der Oberfläche eines Gewebes eines lebenden Organismus sowie ein Verfahren zur Bestimmung von Eigenschaften von Gewebeteilen eines lebenden Organismus.

Der Gegenstand der vorliegenden Erfindung ist insbesondere zur nicht-invasiven Bestimmung geeignet. Unter nicht-invasiven Bestimmungen werden erfindungsgemäß Verfahren verstanden, in deren Verlauf keine körperfremden Materialien durch die Oberfläche in das Gewebe eindringen. Die erfindungsgemäßen Gegenstände sind besonders geeignet zur Langzeitverwendung ohne Beeinträchtigung des Wohlbefindens.

Das erfindungsgemäße System enthält eine optoelektronische Meßvorrichtung, in der die eigentlichen Meßvorgänge stattfinden. Prinzipiell kann im erfindungsgemäßen System jede optoelektronische Meßvorrichtung des Standes der Technik eingesetzt werden, wenn ihre äußere Form auf die erfindungsgemäße Befestigungsvorrichtung abgestimmt ist. Die Erfordernisse hierfür werden später geschildert. Meßvorrichtungen für die Bestimmung verschiedenster Eigenschaften sind beschrieben. Hierzu gehören beispielsweise die Bestimmung struktureller Eigenschaften, z. B. des inneren Aufbaus des Gewebes, jedoch auch die Eigenschaften inhaltlicher Art, z. B. die Anwesenheit und Konzentration von bestimmten Stoffen in dem lebenden Organismus. Als Meßvorrichtung sind besonders bevorzugt Vorrichtungen zur Messung von Analyten in Blut, ganz besonders bevorzugt von Glukose. Bei Gewebe-teilen handelt es sich insbesondere um Kompartimente des Organismus, welche sich innerhalb weniger Zentimeter, insbesondere innerhalb ca. 2 cm, gemessen von der Oberfläche des lebenden Organismus, befinden. Hierzu zählen auch Blutgefäße. Eine besonders bevorzugte optoelektronische Meßvorrichtung ist die in WO 94/10901 beschriebene Vorrichtung.

Im Folgenden wird die Oberfläche des Gewebes, die auf einer ersten Oberfläche (11) der Meßvorrichtung (10) aufliegt, als erste Oberfläche bezeichnet. Als zweite Oberfläche wird eine hierzu lateral benachbarte Oberfläche des Gewebes bezeichnet, an welcher eine auf das Gewebe hin gerichtete (zweite) Oberfläche (21) der Befestigungsvorrichtung (20) befestigt werden kann.

Die optoelektronische Meßvorrichtung weist bevorzugt eine ebene erste Oberfläche (11) auf, die auf die erste Oberfläche des Gewebes gerichtet werden kann. Die erste Oberfläche kann flach, jedoch auch der Anatomie der jeweiligen Oberfläche (z. B. Körperwölbungen) angepaßt sein. Diese Oberfläche (11) enthält mindestens einen Lichtsender und mindestens einen Lichtempfänger, die so angeordnet sind, daß das von dem Lichtsender ausgestrahlte und in dem Gewebe gestreute Licht im Lichtempfänger detektiert werden kann. Die Erfindung bezieht sich insbesondere auf Meßanordnungen, bei denen der Detektionsort an der gleichen Grenzfläche liegt wie der Einstrahlungsort (d. h. Messung in Reflexion im Gegensatz zu Messung in Transmission, bei der sich Einstrahlungsort und Detektionsort auf gegenüberliegenden Grenzflächen des Gewebes befinden).

Ein wesentlicher Bestandteil des erfindungsgemäßen Systems ist eine Befestigungsvorrichtung (20), mit der die Meßvorrichtung (10) auf einer Oberfläche des Gewebes fixiert und von der sie auch zerstörungsfrei wieder gelöst werden kann. Die Befestigungsvorrichtung weist zur zuverlässigen Fixierung der Meßvorrichtung Bauelemente auf, die in ihrer Form auf Bauelemente der Meßvorrichtung adaptiert sind. Im erfindungsgemäßen Sinne sind alle Bauelementepaare geeignet, die ein reproduzierbares Entfernen und Anbringen der Probe mit hoher Positioniergenauigkeit erlauben. Unter reproduzierbar wird verstanden, daß die Meßvorrichtung mehrmals hintereinander entfernt und angebracht werden kann, ohne daß Lichtempfänger und Lichtsender auf unterschiedliche Bereiche des Meßfenster gerichtet sind. Beispiele für solche Bauelemente sind Bajonettverschlüsse, Schraubverschlüsse mit Anschlag bzw. Einrastung oder Klemmverschlüsse und im Prinzip alle Arten von Flanschsystemen (auch solche, die auf Pressung beruhen). Besonders bevorzugt sind jedoch Bauelemente, die ohne Drehbewegung auskommen. Es hat sich nämlich erwiesen, daß durch Drehen der Meßvorrichtung ein Verschieben von Haut, die an der ebenen Oberfläche der Meßvorrichtung anliegt, bewirkt wird, und diese Mitführung der Haut die Reproduzierbarkeit der Anbringung der Meßvorrichtung im Hinblick auf das Gewebe reduziert. Besonders bevorzugt sind daher Bauelemente, die ein in Richtung auf die Oberfläche der Haut senkrechtes Einfügen der Meßvorrichtung in die Befestigungsvorrichtung erlauben. Besonders bevorzugt sind Bauelemente, die auch noch eine optische oder akustische Kontrolle für korrekte Fixierung beinhalten. In der Regel sind hierfür die sogenannten Schnappverschlüsse geeignet. Hierfür werden in der Regel zwei oder mehr Schnappverschlüsse benutzt, wovon einer auch als Führungshilfe, z. B. ein Drehpunkt oder Scharnier, ausgebildet sein kann. Als ganz besonders günstig hat es sich erwiesen Schnappverschlüsse zu verwenden, welche durch leichten, lateralen, nicht auf das Gewebe gerichteten Druck auf das meßvorrichtungsseitige Bauelement zu öffnen sind.

Die Befestigungsvorrichtung enthält erfindungsgemäß bevorzugt ein welches und ein festes Material.

Das befestigungsvorrichtungsseitige Bauelement ist bevorzugt an dem festen Material der Befestigungsvorrichtung angebracht. Besonders bevorzugt ist es aus einem Material ausgebildet, das viele Öffnungs- und Schließvorgänge ohne Funktionseinbußen übersteht. Es kann, muß aber nicht, aus demselben Material gefertigt sein wie die übrige Befestigungsvorrichtung. Es kann sich beispielsweise um einen Haken handeln. Das meßvorrichtungsseitige Bauelement kann gefedert sein. Beispielsweise kann es sich um einen Haken handeln, der durch eine Feder gedrückt in den befestigungsseitigen Haken eingreift. In einer anderen Ausführungsform handelt es sich um einen einfachen Klemmverschluß ohne Feder. Aufgrund der erfindungsgemäßen Gestaltung kann die Meßvorrichtung nur in vorbestimmter und eindeutiger Orientierung in der Befestigungsvorrichtung fixiert werden.

Das weiche und das feste Material umgeben das Meßfenster, durch welches die nicht-invasive Bestimmung vorgenommen werden kann. In einer bevorzugten Ausführungsform ist die Befestigungsvorrichtung daher aus einem inneren, im wesentlichen ringförmigen und im Vergleich zu der Erstreckung auf der Oberfläche des Gewebes relativ schmalen festen Bauteil, und einem im Vergleich hierzu relativ breiten äußeren Rand aus dem weichen Material, aufgebaut. Hierdurch ergibt sich eine im wesentlichen ringförmige Befestigungsvorrichtung, mit einem inneren und äußeren Durchmesser, der an seinem inneren Teil so gestaltet ist, daß die optoelektronische Meßvorrichtung leicht und reproduzierbar im inneren Ring zu befestigen bzw. von diesem Ring zu lösen und robust (insbesondere bei Körperbewegungen) fixiert ist. Der innere Ring ist bevorzugt aus einem harten Kunststoff gefertigt, z. B. aus Delrin®. Der äußere breite Rand ist aus einem weichen Material gefertigt, z. B. in Bezug auf die Weichheit aus silikonähnlichen Materialien, besonders bevorzugt einem thermoplastischen Kunststoff, wie Polyurethan, so daß er sich der Form der zweiten Oberfläche des Gewebes anpassen kann.

Das innere und äußere Material der Befestigungsvorrichtung sind so miteinander verbunden, daß sie während des normalen Gebrauches, d. h. während der Tragezeit der Meßvorrichtung in der Befestigungsvorrichtung und beim Anbringen und Abnehmen der Meßvorrichtung sich nicht voneinander lösen und verdrehen können. Da feste Material ist bevorzugt so in das weiche Material eingearbeitet, daß es möglichst nur an den Stellen der Befestigungsvorrichtung zu sehen ist, die mit der Meßvorrichtung in Berührung kommen können. Inbesondere bedeckt das weiche Material im wesentlichen die gesamte Oberfläche der Befestigungsvorrichtung, die zur Befestigung der Befestigungsvorrichtung auf der Gewebeoberfläche gedacht ist.

Die Befestigungsvorrichtung dient zusätzlich zu ihrer Befestigungsfunktion auch zur Abschirmung der ersten Oberfläche des Gewebes vor ungewünschtem Umgebungslicht. Aus diesem Grund sind die Materialien, aus denen die Vorrichtung aufgebaut ist, für Licht undurchlässig insbesondere für Licht, welches für die Bestimmung der Eigenschaften verwendet wird. Dies kann beispielsweise geschehen durch Pigmentierung der Materialien mit Stoffen, die Licht absorbieren.

Als Meßfenster wird eine Stelle in der Befestigungsvorrichtung bezeichnet, durch welche die erste Oberfläche des Organismus mit Licht aus der Meßvorrichtung bestrahlt, und durch die gestreutes Licht aus dem Gewebe auf die Meßvorrichtung fallen kann. Die wesentliche Bedingung ist daher, daß das Meßfenster für eingestrahltes und gestreutes Licht transparent ist. In einer besonders einfachen und daher bevorzugten Ausführungsform besteht das Meßfenster in einer Öffnung in dem Material der Befestigungsvorrichtung, so daß die ebene Oberfläche der Meßvorrichtung mit der Oberfläche des Gewebes direkt in Kontakt kommen kann, wenn die Meßvorrichtung in der Befestigungsvorrichtung fixiert ist.

Es können auch mehrere, nebeneinander liegender Meßfenster vorgesehen sein, z. B. je ein Meßfenster für jeden Lichtsender und jeden Lichtempfänger.

Die Befestigung der Befestigungsvorrichtung mit ihrer Oberfläche (21) auf der zweiten Oberfläche des Gewebes wird bevorzugt durch einen ersten Kleber bewerkstelligt. Es hat sich hierbei als besonders vorteilhaft herausgestellt, ein doppelseitiges Klebeband zu verwenden. Solche Klebebänder sind bekannt. Beispielsweise ist das doppelseitige Polyesterklebeband der Firma Adhesives Research (Bestell-Nr. AR 8254) auf Acrylkleberbasis geeignet.

Besonders geeignet sind Klebebänder, die eine Fixierung der Befestigungsvorrichtung auf dem Gewebe, z. B. der menschlichen Haut, über einen Zeitraum von mehreren, bevorzugt mindestens 3 Tagen erlauben. Hierzu ist der Kleber bevorzugt feuchtigkeitsaufnehmend. Während der genannten Tragezeiten bildet sich nämlich auf der Haut normalerweise Feuchtigkeit (z. B. Schweiß), die bei unzureichender Abführung auf der Haut verbleibt und zu Hautirritationen führen kann. Der Kleber kann in einer anderen Ausführungsform auch feuchtigkeitsdurchlässige Eigenschaften besitzen. Hierzu ist es sinnvoll, daß der weiche Teil der Befestigungsvorrichtung ebenfalls feuchtigkeitsdurchlässig ist. Dies ist realisierbar z. B. durch Einsatz perforierter Materialien. Der Kleber sollte keine allergieauslösenden Substanzen beinhalten.

Es ist außerdem bevorzugt, daß der eingesetzte Kleber nach der gesamten Tragezeit sich leichter von der Oberfläche des Gewebes als von der zur Fixierung verwendeten ebenen Oberfläche der Befestigungsvorrichtung lösen kann. Dies ist bei dem oben beispielhaft genannten Kleber gegeben.

Der Kleber, welcher zur Befestigung der Befestigungsvorrichtung auf der Gewebeoberfläche verwendet wird, sollte außerdem lichtabsorbierende Eigenschaften aufweisen. Es hat sich nämlich herausgestellt, daß vermieden werden muß, daß Umgebungslicht auf die zu der Befestigungsvorrichtung direkt benachbarten Hautareale fällt. Du da einfallende Licht kann nach Eintritt ins Gewebe und Durchstrahlung der Haut Störsignale an den Lichtempfängern hervorrufen. Es ist besonders bevorzugt, daß der Außendurchmesser des Klebebandes etwas größer ist als der Durchmesser der Befestigungsvorrichtung. Der Teil des Klebebandes, der nicht von der Befestigungsvorrichtung abgedeckt ist, wird bevorzugt mit einem lichtundurchlässigen Film versehen oder mit einem lichtundurchlässigen Farbstoff imprägniert, um ein Eindringen von Außenlicht in den Meßbereich zu verhindern oder zumindest stark zu reduzieren.

In einer besonderen Ausführungsform enthält das erfindungsgemäße System einen weiteren (zweiten) Kleber, z. B. in Form eines doppelseitigen Klebebandes, der auf der ebenen Oberfläche der Meßvorrichtung angebracht ist. Dieser Kleber stellt nach Einbringung der Meßvorrichtung in die Befestigungsvorrichtung das eigentliche Interface zwischen der Oberfläche und der Meßvorrichtung dar. Da sowohl das von der Meßvorrichtung emittierte als auch das aus dem Gewebe gestreute Licht durch diesen Kleber hindurchtreten müssen, muß das Material eine möglichst hohe Transmission für die verwendeten Wellenlängenbereiche des Lichts aufweisen. Es sollte möglichst transparent sein. Darüber hinaus hat es sich als äußerst zweckmäßig erwiesen, wenn der Kleber keine Lichtleitereigenschaften aufweist. Dies bedeutet, daß der Kleber so ausgewählt werden sollte, daß möglichst keine oder nur eine sehr geringe Reflexion an den Innenflächen des Klebers stattfindet. Insbesondere sollte eine laterale Lichtleitung durch Reflexionen an den Innenflächen oder Inhomogenitäten im Kleber vermieden werden. Diese Maßnahmen dienen dazu, ein Übersprechen (cross talk) zwischen Sender und Empfänger über den Kleber zu verhindern. Der Kleber wird so ausgesucht, daß ein möglichst geringer Lichtverlust beim vertikalen Durchtritt des Lichts stattfindet.

Darüber hinaus hat es sich jedoch auch noch aus anderem Grunde als besonders vorteilhaft erwiesen, die erste Oberfläche der Meßvorrichtung fest mit der ersten Oberfläche des Gewebes zu verbinden. Wichtig ist ein inniger Kontakt zwischen den Oberflächen. Der Kleber (12) gewährleistet das Einhalten eines gleichmäßigen Abstandes zwischen den Gewebekompartimenten und der Meßvorrichtung. Darüber hinaus wird die horizontale Verschiebung der Gewebeoberfläche relativ zu der Meßvorrichtung, z. B. bei Bewegungen, weitgehend reduziert. Die Verwendung eines Klebers hat außerdem den Vorteil, daß die Oberfläche des Gewebes eben bleibt. Unterschiedlich große Luftstrecken zwischen Haut und Meßvorrichtung, wie sie beispielsweise bei einer Gänsehaut vorkommen können, werden hierdurch vermieden. Die oben genannten Komplikationen bei der nicht-invasiven Bestimmung von Eigenschaften an einem lebendem Organismus wurden bislang offensichtlich dadurch umgangen, daß manche der verwendeten Meßvorrichtungen mit erhöhtem Druck gegen die Obenfläche gepreßt wurden. Die Anwendung von Druck auf Gewebe kann jedoch einige Gewebeeigenschaften, z. B. Durchblutung, verändern, weshalb die bekannten Vorrichtungen für eine Langzeitanwendung nicht geeignet waren. Dadurch, daß die erfindungsgemäße Vorrichtung bei der Verwendung von Klebern ohne Anwendung von Druck (außer bei der Anbringung der Vorrichtung) auskommt, werden Langzeitanwendungen für den Organismus möglich.

Auch für diesen Kleber (12) ist es bevorzugt, daß er feuchtigkeitsaufnehmende Eigenschaften aufweist, die einen möglichst geringen Einfluß auf die optischen Eigenschaften haben. Eine Tragedauer der Meßvorrichtung von 4 Stunden oder mehr ohne Unterbrechung wird hierdurch möglich.

Die Klebekraft des zweiten Klebers (12) sollte im Vergleich zum ersten Kleber (22) deutlich geringer sein. Während der erste Kleber praktisch die gesamte Kraft zur Fixierung des Systems an dem Gewebe aufbringen muß, stehen die Befestigungseigenschaften beim zweiten Kleber im Hintergrund. Er sollte nur soviel Klebekraft aufbringen, daß der gute Kontakt zwischen Gewebe und der Meßvorrichtung erhalten bleibt und die Vermeidung der Verschiebung der Oberfläche gegen die Meßanordnung gewährleistet ist. Darüber hinaus ist eine im Vergleich zum ersten Kleber geringere Klebekraft bevorzugt, da dann eine Entnahme der Meßvorrichtung von der Befestigungsvorrichtung und der Oberfläche des Gewebes möglich ist, ohne daß auch die Befestigungsvorrichtung von der Oberfläche gelöst wird. Geeignete Kleber sind beispielsweise Acrylkleber. Als Beispiel kann klares Polyurethanklebeband der Firma Adhesives Research (Bestell-Nr. AR 8133) auf Basis des "Hydro absorbent™ adhesive systems" mit wasseraufnehmenden und wasserdampfdurchlässigen Eigenschaften verwendet werden. Dieses Klebeband ist zwar nur einseitig klebend beschichtet, kann jedoch auf Antrage bei der Firma Adhesive Research auch zweiseitig klebend erhalten werden. Weiterhin gehört es zum Fachwissen, dieses Klebeband in ein zweiseitig klebendes Band umzuwandeln. Es ist daher weiterhin bevorzugt, daß der zweite Kleber sich besser von der Haut löst als von der Oberfläche der Meßvorrichtung.

Ein weiterer Kleber, der sich sowohl für eine Befestigung der Befestigungsvorrichtung als auch der Meßvorrichtung auf Haut bewährt hat, ist bei der Firma Lohmann unter der Bezeichnung "Duplomed 2806" erhältlich. Dieser Kleber bzw. dieses doppelseitige Klebeband ist sowohl feuchtigkeitsaufnehmend als auch feuchtigkeitsdurchlässig. Senkrecht zur Ebene des Klebebandes ist dieses ausreichend lichtdurchlässig, um es zu durchstrahlen und von dem untersuchten Gewebeteil gestreute Strahlung hindurchzulassen. Lateral, d.h., in der Ebene des Klebebandes besitzt dieses eine ausreichend geringe Lichtdurchlässigkeit, um ein Übersprechen zwischen Sender und Empfänger zu vermeiden. Ein Klebeband, das ein Übersprechen noch wirksamer unterdrücken soll, kann in seiner Funktionsweise noch durch Einfärbung mit Pigmenten verbessert werden.

Vorteil der vorliegenden Erfindung ist die Möglichkeit, die Meßvorrichtung positionsgenau relativ zu der Oberfläche des Gewebes zu fixieren. Hierunter sind sowohl Genauigkeit in vertikaler Richtung als auch in horizontaler Richtung zu verstehen. Die Genauigkeit kann besser als ± 0.2 mm, bevorzugt ± 0.1 mm erreichen. Auch kann eine Verbesserung der Positionsgenauigkeit in Bezug auf eine Drehachse senkrecht zur Oberfläche realisiert werden. Die Positionsgenauigkeit ist von besonderer Bedeutung, da festgestellt wurde, daß die Reproduzierbarkeit wegen der Inhomogenität von Gewebe durch eine genaue und reproduzierbare Positionierung der Meßvorrichtung erheblich verbessert wird. Dadurch sind erheblich weniger Neukalibrierungen der Meßvorrichtung erforderlich.

Darüber hinaus ist es erfindungsgemäß möglich, eine effektive Verhinderung von Lichteinfall zwischen Befestigungsvorrichtung und Meßvorrichtung zu gewährleisten, ohne daß die Entfernbarkeit der Meßvorrichtung darunter leidet.

In einer bevorzugten Ausführungsform ist die äußere, nicht auf das Gewebe zu gerichtete Form des System so gestaltet, daß möglichst wenige Ecken und Kanten entstehen. Eine flache Bauweise ist daher besonders bevorzugt.

Die Meßvorrichtung muß nicht alle für die Durchführung der Bestimmung erforderlichen Bauteile selbst enthalten. Es ist sogar bevorzugt, daß manche größere Bauteile (z. B. zur Auswertung der Messungen) in Vorrichtungen untergebracht sind, die mit der Meßvorrichtung (10) (z. B. über ein Versorgungskabel (17)) verbunden sind.

Ebenfalls Gegenstand der Erfindung ist die Vorrichtung zur Befestigung einer optoelektronischen Meßvorrichtung an der Oberfläche eines Gewebes eines lebenden Organismus enthaltend einen Formkörper (20), der ein Meßfenster (28) umgibt, und der mindestens ein Bauelement (26) zur reproduzierbaren Fixierung der Meßvorrichtung aufweist.

Bevorzugt weist die erfindungsgemäße Befestigungsvorrichtung auf einer ebenen Oberfläche einen Klebestoff zur Befestigung der Vorrichtung an der Oberfläche des Gewebes auf. Der Kleber hat bevorzugt lichtabsorbierende oder/und feuchtigkeitsaufnehmende oder/und feuchtigkeitsdurchlässige Eigenschaften. Das weiche und das feste Material haben bevorzugt lichtabsorbierende Eigenschaften. Darüber hinaus hat das weiche Material zusätzlich bevorzugt feuchtigkeitsaufnehmende oder/und feuchtigkeitsdurchlässige Eigenschaften. Weitere bevorzugte Ausführungen sind oben für das erfindungsgemäße System beschrieben.

Ein weiterer Gegenstand der Erfindung ist ein Verfahren zur, bevorzugt nicht-invasiven, Bestimmung von Eigenschaften von Gewebeteilen eines lebenden Organismus durch
a) Anbringen einer Vorrichtung (20), die für Licht undurchlässig ist, und die ein Meßfenster (28) umgibt, und die mindestens ein Bauelement (26) zur reproduzierbaren Fixierung einer Meßvorrichtung (10) aufweist, auf einer Oberfläche des Gewebes,
b) Fixierung der Meßvorrichtung (10) in der Vorrichtung (20), so daß die Vorrichtung (10) durch das Meßfenster (28) auf eine Oberfläche des Gewebes gerichtet ist,
c) Bestrahlung des Gewebes mit Licht der Meßvorrichtung,
d) Detektion von in dem Gewebe gestreuten Licht der Meßvorrichtung zum Erhalt eines Meßwertes,
e) Wiederholung der Schritte c) und d) während eines Zeitraumes von mindestens 4 Stunden.

Bevorzugte Ausführungen ergeben sich durch Einsatz des erfindungsgemäßen Systems.

Dieses Verfahren wird dadurch möglich, daß die Meßvorrichtung sehr genau über längere Zeit an einer Stelle der Oberfläche des Gewebes angebracht werden kann. Durch die erfindungsgemäße Verwendung der Kleber werden Hautirritationen vermieden.

Dadurch, daß die Befestigungsvorrichtung bei Entfernung der Meßvorrichtung an der Oberfläche des Gewebes verbleiben kann, ist es möglich, die Meßanordnung, z. B. nach einem eventuell zu Beeinträchtigungen der Meßvorrichtung führenden Vorgang (z. B. Duschen), auf der exakt gleichen Stelle der Oberfläche zu repositionieren.

Hierzu ist es bevorzugt, daß die Meßvorrichtung so von der (ersten) Oberfläche des Gewebes gelöst wird, daß der (zweite) Kleber bei Entnahme der Meßvorrichtung aus der Befestigungsvorrichtung an der Meßvorrichtung verbleibt. Anschließend kann der Kleber auch von der Meßvorrichtung entfernt werden und, bei erneuter Fixierung ein neuer Klebestreifen auf dessen Oberfläche befestigt werden. Wie im Falle von doppelseitigen Klebebändern üblich, wird zum Transport oder zur Aufbewahrung die Oberfläche der Meßvorrichtung, die zur Befestigung auf der Gewebeoberfläche vorgesehen ist, durch ein leicht entfernbares Deckband geschützt, welches kurz vor Anbringen der Vorrichtung abgezogen wird.

Die Befestigungsvorrichtung verbleibt bevorzugt für einen kontinuierlichen Zeitraum von mehreren, bevorzugt mindestens 3 Tagen auf der Oberfläche des Gewebes. Sofern anschließend erneut ein Bestimmungsintervall vorgesehen werden soll, kann eine neue Befestigungsvorrichtung an der gleichen Stelle angebracht werden. Damit die Messung wieder an der im wesentlichen identischen Stelle des Gewebes stattfindet, kann die Position der zu entfernenden Vorrichtung auf der Gewebeoberfläche markiert werden, z. B. am Rande des Meßfensters. Bei der Anbringung der neuen Befestigungsvorrichtung kann auf diese Markierung Bezug genommen werden.

Wenn die Meßvorrichtung nicht in der Befestigungsvorrichtung befestigt ist, kann die Aufnahme (29) der Befestigungsvorrichtung durch ein Ersatzbauteil (Dummy) verschlossen werden, welches im wesentlichen die äußere Form der Meßvorrichtung wiedergibt. Dadurch können das Meßfenster, die Aufnahme und die erste Oberfläche des Gewebes während der Zeit, während der die Meßvorrichtung nicht getragen wird, vor schädigenden Einflüssen (Verschmutzung etc.) geschützt werden.

In den Figuren werden bevorzugte Ausführungsformen für die Befestigungsvorrichtung bzw. die Meßvorrichtung gezeigt.

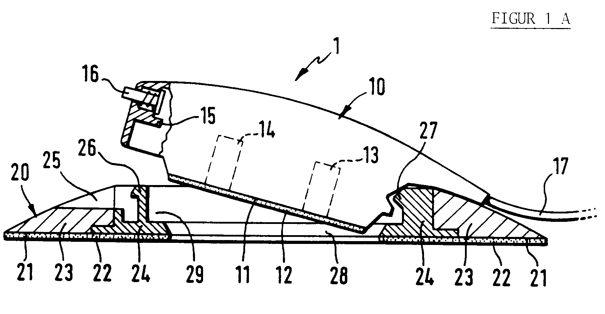

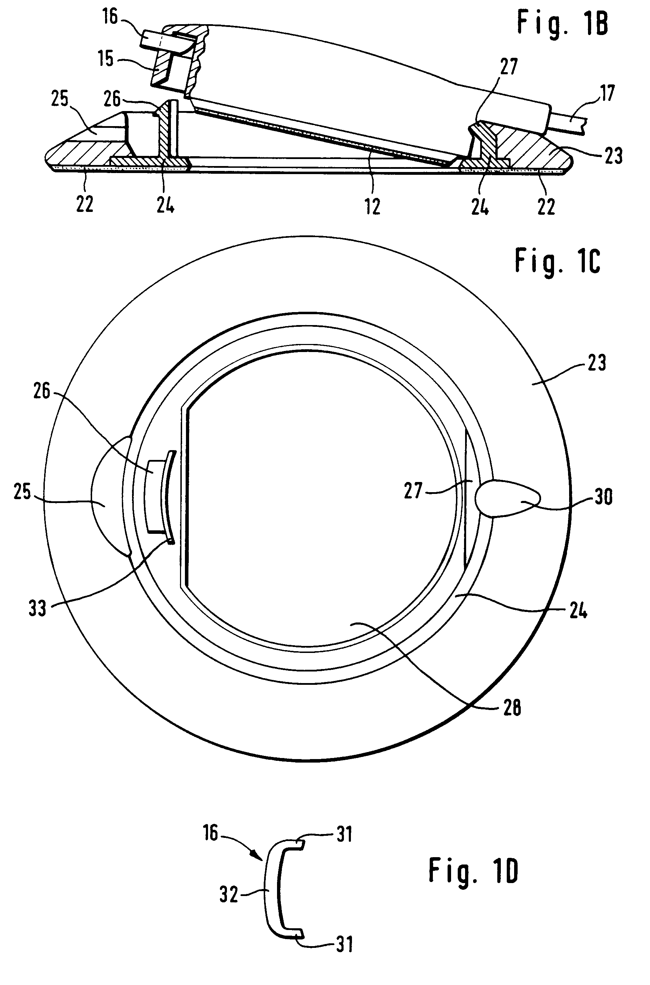

In Figur 1A und 1B ist ein System gezeigt, bei dem die Fixierung der Meßvorrichtung in der Befestigungsvorrichtung mittels eines Schnappverschlusses und einer gegenüberliegenden Führungshilfe bewerkstelligt wird.

In Figur 1A ist das System im Querschnitt in einem Zustand gezeigt, bei dem die Meßvorrichtung mit einer Ausnehmung bereits in eine Führungshilfe (27) der Befestigungsvorrichtung eingreift, der Schnappverschluß auf der gegenüberliegenden Seite jedoch noch nicht zugeschnappt ist. Der Schnappverschlußteil (15) der Meßvorrichtung ist hier federnd gelagert und kann mit Hilfe des Druckknopfes (16) ausgelöst werden.

In Figur 1B ist der Schnappverschlußteil (15) der Meßvorrichtung als Gehäuseteil ausgeführt und der Verschluß kann mit Hilfe eines einfachen, geklemmten, federlosen Druckknopfes (16) gelöst werden. Hierzu werden Meß- und Befestigungsvorrichtung durch lateralen Druck auf das herausragende Verbindungsstück (32) des Druckknopfes (16) gelöst. Hierbei drücken die beiden Stege (31) auf die Überstände (33) des Schnappverschlußteiles (26) der Befestigungsvorrichtung. Dadurch wird die Verhakung des Verschlußteiles (26) und des Verschlußteiles (15) gelöst.

Die Verbindung von Befestigungsvorrichtung und Meßvorrichtung wird so durchgeführt, daß zunächst die Meßvorrichtung (10) in die Führungshilfe (27) an die Befestigungsvorrichtung angelegt wird. Danach wird die Verbindung von Meß- und Befestigungsvorrichtung durch leichten Druck von oben auf die Meßvorrichtung hergestellt. Dabei schnappt der Schnappverschlußteil (15) der Meßvorrichtung unter den Schnappverschlußteil (26) der Befestigungsvorrichtung. Druckknopf (16) gemäß Fig. 1B wird dabei selbsttätig in die Ursprungsposition gedrückt.

In der Ausführungsform nach Fig. 1A behält der Druckknopf (16) aufgrund der Federkraft seine Ursprungsposition bei.

In Figur 1C ist die Befestigungsvorrichtung in Aufsicht gezeigt.

In Figur 1D ist der federlose Klemmverschluß (16) in Obenaufsicht gezeigt, bestehend aus zwei Stegen (31) und einem breiteren, bogenförmigen Verbindungsstück (32).

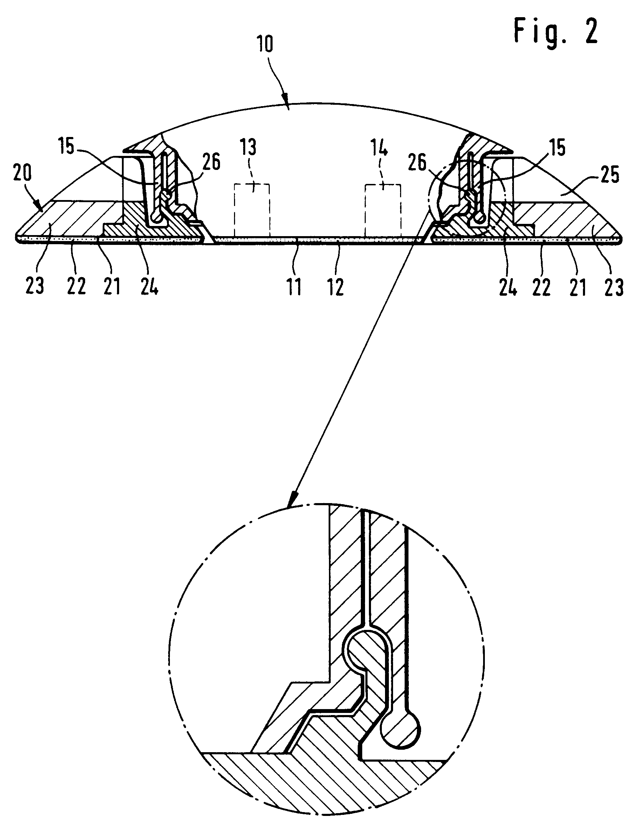

In Figur 2 ist eine Ausführungsform gezeigt, bei der die Fixierung der Meßvorrichtung in der Befestigungsvorrichtung durch einen kreisförmigen äußeren Rand gewährleistet wird, der unter eine an der Meßvorrichtung angebrachte Lasche greift und bei perfektem Sitz in eine Ausnehmung einschnappt. Die im Kreis enthaltene Zeichnung gibt die Details des Bauelementes (26) wieder.

### Bezugszeichenliste

- (1): System
- (10): Meßvorrichtung
- (11): Oberfläche der Meßvorrichtung (erste Oberfläche)
- (12): Kleber auf der ersten Oberfläche von (10)
- (13): Lichtsender
- (14): Lichtempfänger
- (15): Schnappverschlußteil der Meßvorrichtung
- (16): Druckknopf
- (17): Versorgungskabel zur Auswerteeinheit
- (20): Befestigungsvorrichtung
- (21): Oberfläche der Befestigungsvorrichtung (zweite Oberfläche)
- (22): Kleber auf Oberfläche von (21)
- (23): weiches Material
- (24): festes Material
- (25): Ausnehmung um Betätigung von Knopf 16 zu ermöglichen
- (26): Schnappverschlußteil der Befestigungsvorrichtung (Bauelement)
- (27): Nase, Führungshilfe
- (28): Meßfenster
- (29): Aufnahme für die Meßvorrichtung
- (30): Aussparung für Versorgungskabel
- (31): Stege
- (32): Verbindungsstück
- (33): Überstände

## Patentansprüche

1. System zur Bestimmung von Eigenschaften von Gewebeteilen eines lebenden Organismus enthaltend
- eine optoelektronische Meßvorrichtung (10),
- eine Vorrichtung (20), die für Licht undurchlässig ist, und die ein Meßfenster (28) umgibt, durch welches die Bestimmung vorgenommen werden kann und wobei die Vorrichtung mindestens ein Bauelement (26) zur reproduzierbaren, auf eine erste Oberfläche des Gewebes gerichteten, Fixierung der Meßvorrichtung (10) aufweist.

2. System gemäß Anspruch 1, dadurch gekennzeichnet, daß die Vorrichtung (20) eine Oberfläche (21) aufweist, die einen Kleber (22) zur Befestigung der Vorrichtung (20) auf einer zweiten zu der ersten Oberfläche des Gewebes benachbarten Oberfläche des Gewebes aufweist.

3. System gemäß Anspruch 2, dadurch gekennzeichent, daß der Kleber feuchtigkeitsdurchlässig ist.

4. System gemäß Anspruch 2, dadurch gekennzeichnet, daß der Kleber feuchtigkeitsaufnehmende Eigenschaften besitzt.

5. System gemäß Anspruch 2, dadurch gekennzeichnet, daß der Kleber lichtabsorbierende Eigenschaften besitzt.

6. System gemäß Anspruch 1, dadurch gekennzeichnet, daß die Materialien der Vorrichtung (20) das Meßfenster ringförmig umgeben.

7. System gemäß Anspruch 2, dadurch gekennzeichnet, daß der Kleber die Form eines doppelseitigen Klebebandes hat.

8. System gemäß Anspruch 1, dadurch gekennzeichnet, daß es ferner einen Kleber (12) enthält, mit dem eine Oberfläche (11) der Meßvorrichtung (10) an der ersten Oberfläche des Gewebes fixiert werden kann.

9. System gemäß Anspruch 8, dadurch gekennzeichnet, daß der Kleber (12) für Licht, welches bei der Bestimmung benutzt wird, transparent ist.

10. System gemäß einem der Ansprüche 8 oder 9, dadurch gekennzeichnet, daß der Kleber (12) keine oder nur geringe Lichtleitereigenschaften aufweist.

11. System gemäß einem der Ansprüche 8, 9 oder 10, dadurch gekennzeichnet, daß der Kleber (12) feuchtigkeitsaufnehmende Eigenschaften besitzt.

12. System gemäß Anspruch 1, dadurch gekennzeichnet, daß die Bestimmung nicht-invasiv ist.

13. System gemäß Anspruch 1, dadurch gekennzeichnet, daß die Vorrichtung ein weiches und ein festes Material enthält, wobei das Bauelement bzw. die Bauelemente (26) sich an dem festen Material befinden.

14. System gemäß Anspruch 1, dadurch gekennzeichnet, daß die Oberfläche (11) eben ist.

15. Vorrichtung zur Befestigung einer optoelektronischen Meßvorrichtung an der Oberfläche eines Gewebes eines lebenden Organismus enthaltend einen Formkörper, der ein Meßfenster (28) umgibt und mindestens ein Bauelement (26) zur reproduzierbaren Fixierung der Meßvorrichtung aufweist.

16. Vorrichtung gemäß Anspruch 15, dadurch gekennzeichnet, daß sie auf einer Oberfläche (21) einen Kleber (22) zur Befestigung der Vorrichtung an der Oberfläche des Gewebes aufweist.

17. Vorrichtung gemäß Anspruch 16, dadurch gekennzeichnet, daß der Kleber (22) lichtabsorbierende oder/und feuchtigkeitsdurchlässige oder/und feuchtigkeitsaufnehmende Eigenschaften aufweist.

18. Vorrichtung gemäß Anspruch 16, dadurch gekennzeichnet, daß sie ein weiches und ein festes Material mit lichtabsorbierenden Eigenschaften enthält.

19. Vorrichtung gemäß Anspruch 15, dadurch gekennzeichnet, daß sie eine Aufnahme (29) aufweist, die durch ein Ersatzbauteil der Meßvorrichtung verschlossen ist.

20. Verfahren zur Bestimmung von Eigenschaften von Gewebeteilen eines lebenden Organismus durch
a) Anbringen einer Vorrichtung (20), die für Licht undurchlässig ist und die ein Meßfenster (28) umgibt, und die mindestens ein Bauelement (26) zur reproduzierbaren Fixierung einer Meßvorrichtung (10) auf einer ersten Oberfläche des Gewebes aufweist,
b) Fixierung der Meßvorrichtung (10) in der Vorrichtung (20), so daß die Vorrichtung (10) durch das Meßfenster (28) auf eine erste Oberfläche des Gewebes gerichtet ist,
c) Bestrahlung des Gewebes mit Licht durch die Meßvorrichtung,
d) Detektion von in dem Gewebe gestreuten Licht durch die Meßvorrichtung zum Erhalt eines Meßwertes,
e) Wiederholung der Schritte c) und d) während eines Zeitraumes von mindestens 4 Stunden.

21. Verfahren gemäß Anspruch 20, dadurch gekennzeichnet, daß die Meßvorrichtung (10) nach ein- oder mehrmaliger Durchführung der Schritte c) und d) aus der Befestigungsvorrichtung (20) entnommen wird, wobei die Vorrichtung (20) auf der ersten Oberfläche verbleibt und anschließend die Meßvorrichtung (10) wieder in der Befestigungsvorrichtung (20) fixiert wird.

22. Verfahren gemäß Anspruch 21, dadurch gekennzeichnet, daß die Meßvorrichtung an ihrer Oberfläche (11) einen Kleber (12) aufweist, der bei Entnahme der Meßvorrichtung (10) aus der Befestigungsvorrichtung (20) an der Meßvorrichtung (10) verbleibt.

23. Verfahren gemäß Anspruch 22, dadurch gekennzeichnet, daß der Kleber (12) nach Entnahme der Meßvorrichtung (10) von der Oberfläche (11) entfernt wird.
